# EUROPEAN PATENT APPLICATION

(11) **EP 4 710 939 A1**
(43) Date of publication of application: **18.03.2026**
(21) Application number: 24803051.2
(22) Date of filing: 09.05.2024
(51) Int. Cl.: A61K 39/395, C07K 16/46, A61P 35/00, A61P 35/04

(54) **COMBINED THERAPY DRUG COMPRISING ANTI-HER2 BISPECIFIC ANTIBODY AND USE THEREOF**

(30) Priority: 09.05.2023 CN 202310514172
(71) Applicant: Jiangsu Alphamab Biopharmaceuticals Co., Ltd., Jiangsu 215000 (CN); Shanghai JMT-Bio Technology Co., Ltd., Shanghai 200032 (CN)
(72) Inventor: HUANG, Wei, Shanghai 200032 (CN); XIANG, Silong, Shanghai 200032 (CN); SHE, Fenglin, Shanghai 200032 (CN); ZHAO, Yuqing, Shanghai 200032 (CN); ZHANG, Xiaojun, Shanghai 200032 (CN); WANG, Caixia, Shanghai 200032 (CN); WU, Guohua, Shanghai 200032 (CN)
(74) Representative: Latscha Schöllhorn Partner AG
(86) International application number: PCT/CN2024/091943
(87) International publication number: WO 2024/230772

(57) **Abstract**

A combined therapeutic medicament comprising an anti-HER2 bispecific antibody and a use thereof, especially a use of an anti-HER2 bispecific antibody combined with a docetaxel albumin composition in the preparation of a medicament for treating advanced malignant solid tumors.

## Description

### TECHNICAL FIELD

The present disclosure relates to the field of biopharmaceuticals and more specifically to a combination-therapy medicament comprising an anti-HER2 bispecific antibody and use thereof, particularly use of the anti-HER2 bispecific antibody in combination with a docetaxel albumin composition in the preparation of a medicament for treating advanced malignant solid tumors.

### BACKGROUND

According to the latest data released by the International Agency for Research on Cancer, there were as many as 2.26 million new cases of breast cancer and 684,000 deaths from breast cancer worldwide in 2020. Breast cancer has overtaken lung cancer as the most commonly diagnosed cancer worldwide, and it is the leading cause of cancer mortalities among women. In China, there were 416,000 new cases of breast cancer, which accounted for 18.4% of the global number of new cases, and 117,000 deaths, which accounted for 17.1% of the global number of deaths from breast cancer, and these numbers are growing year by year. Breast cancer poses a serious threat to the lives and health of women in China. As a result, it has become one of the focuses in China's tumor prevention and treatment efforts.

Breast cancer is classified into different histopathological subtypes based on the expression of estrogen receptor/progestogen receptor and human epidermal growth factor receptor 2 (HER2). HER2-positive breast cancer cases account for approximately 15-20% of all breast cancer cases. In China, however, the proportion of HER2-positive breast cancer cases is higher (20-30%), compared to Western countries. HER2-positive breast cancer is a more aggressive subtype and tends to occur in younger people. Compared to hormone receptor-positive/HER2-negative and triple-negative breast cancer types, HER2-positive breast cancer is more frequently diagnosed at stages III-IV in new cases and presents with a higher histological grade, and most newly diagnosed patients are under the age of 65 years. In China, several drugs have been approved for the treatment of HER2-positive breast cancer, including trastuzumab, pertuzumab, lapatinib, neratinib, pyrotinib, and ado-trastuzumab emtansine (T-DM1). These drugs are used throughout the treatment of HER2-positive breast cancer, including neoadjuvant therapy, postoperative adjuvant therapy, and systemic therapy for recurrent or metastatic disease. Nevertheless, treatments for recurrent or metastatic breast cancer are mainly used for palliative and salvage purposes; there is room for further improvement.

In China, approximately 20% of patients are initially diagnosed with advanced (stage III/IV) breast cancer. In addition, approximately 10% of subjects with early HER2-positive breast cancer experience disease recurrence within 3 years of undergoing radical surgery. The trastuzumab + pertuzumab + docetaxel first-line therapy has been shown to significantly improve the PFS and OS of patients with HER2-positive metastatic breast cancer. However, most subjects still experience disease progression after treatment. This underscores the need for new and more effective treatment options. In addition, the trastuzumab + pertuzumab + docetaxel regimen requires cyclic administration until disease progression or intolerance occurs, and in each cycle (3 weeks), it takes 30-60 minutes to intravenously infuse a dose of pertuzumab (it takes 60 minutes to intravenously infuse a loading dose) and 30-90 minutes to infuse a dose of trastuzumab (it takes 90 minutes to intravenously infuse a loading dose), and an observation period of 30-60 minutes is recommended between administrations of the two. The prolonged and repetitive intravenous infusion process not only reduces treatment convenience but also extends patients' hospital stays for treatment, increasing the risk of hospital-acquired infections and placing a greater strain on healthcare resources. To address these issues, further solutions are needed. In summary, there is an urgent need for a more effective, safer, and more convenient first-line treatment option for HER2-positive breast cancer.

Docetaxel is a semi-synthetic product obtained from a non-cytotoxic precursor (10-deacetylbaccatin III) extracted from yew needles. It is a paclitaxel analog and exhibits stronger antitumor activity than paclitaxel does. Docetaxel is poorly soluble in water. Existing commercially available docetaxel formulations are common injections. The first docetaxel injection was developed by Sanofi under the trade name TAXOTERE^{®}. This injection is routinely administered once every 3 weeks at a dose of 75 mg/m² by intravenous infusion over 1 h. However, docetaxel injections, with TAXOTERE^{®} being a representative example, contain ethanol and Tween 80, which may cause severe allergic reactions. This necessitates pretreatment with dexamethasone, leading to poor patient compliance. In addition, ethanol can affect the central nervous system; therefore, a reduced infusion rate is required to mitigate toxicity-related symptoms.

### SUMMARY

To address the defects of the prior art, the present disclosure provides a combination-therapy medicament for treating cancer. The medicament combines an anti-HER2 bispecific antibody and a docetaxel albumin composition and is used for treating HER2-positive recurrent or metastatic breast cancer. The medicament has superior antitumor efficacy compared to existing standard treatment regimens, has good overall safety, and is more convenient to administer. The medicament can provide such patients with a first-line treatment option that has superior efficacy and good safety and is more convenient to administer.

In a first aspect, the present disclosure provides use of an anti-HER2 antibody and a second therapeutic agent in the preparation of a medicament for treating cancer, wherein the second therapeutic agent is a docetaxel albumin composition.

In a second aspect, the present disclosure provides use of an anti-HER2 antibody in the preparation of a medicament for enhancing efficacy of a second therapeutic agent against cancer, wherein the second therapeutic agent is a docetaxel albumin composition.

In a third aspect, the present disclosure provides a method for treating cancer with an anti-HER2 antibody and a second therapeutic agent, wherein the second therapeutic agent is a docetaxel albumin composition.

In a fourth aspect, the present disclosure provides a pharmaceutical composition or kit or kit of parts for treating cancer, comprising an anti-HER2 antibody and a second therapeutic agent, wherein the second therapeutic agent is a docetaxel albumin composition.

Regarding the above aspects:
In some embodiments, a combination of the anti-HER2 antibody and the second therapeutic agent is synergistic in the treatment of cancer.

In some embodiments, the anti-HER2 antibody is an anti-HER2 bispecific antibody. In some embodiments, the anti-HER2 antibody is a bispecific antibody targeting different epitopes of human HER2 protein.

In some embodiments, the anti-HER2 antibody is a bispecific antibody targeting different epitopes of human HER2 protein, wherein the different epitopes of the HER2 protein are domain II and domain IV thereof.

In some embodiments, the anti-HER2 antibody is a recombinant humanized antibody. In some embodiments, the anti-HER2 antibody comprises two common light chains with identical sequences. In some embodiments, the common light chains comprise a CDR-L1 set forth in SEQ ID NO: 1, a CDR-L2 set forth in SEQ ID NO: 2, and a CDR-L3 set forth in SEQ ID NO: 3. In some embodiments, the common light chains comprise a light chain variable region set forth in SEQ ID NO: 4, or the light chain variable region thereof consists of the amino acid sequence set forth in SEQ ID NO: 4. In some embodiments, the common light chains comprise or consist of an amino acid sequence set forth in SEQ ID NO: 5.

In some embodiments, the common light chains of the anti-HER2 antibody are of a Kappa subtype.

In some embodiments, the anti-HER2 antibody comprises two heavy chains with different sequences. In some embodiments, the heavy chains comprise a heavy chain I targeting domain II of the HER2 protein. In some embodiments, the heavy chain I comprises a CDR-H1-1 set forth in SEQ ID NO: 6, a CDR-H1-2 set forth in SEQ ID NO: 7, and a CDR-H1-3 set forth in SEQ ID NO: 8. In some embodiments, the heavy chain I comprises a heavy chain I variable region set forth in SEQ ID NO: 9, or the heavy chain I variable region consists of the amino acid sequence set forth in SEQ ID NO: 9. In some embodiments, the heavy chain I comprises or consists of an amino acid sequence set forth in SEQ ID NO: 10 or SEQ ID NO: 16.

In some embodiments, the anti-HER2 antibody comprises two heavy chains with different sequences. In some embodiments, the heavy chains comprise a heavy chain II targeting domain IV of the HER2 protein. In some embodiments, the heavy chain II comprises a CDR-H2-1 set forth in SEQ ID NO: 11, a CDR-H2-2 set forth in SEQ ID NO: 12, and a CDR-H2-3 set forth in SEQ ID NO: 13. In some embodiments, the heavy chain II comprises a heavy chain II variable region set forth in SEQ ID NO: 14, or the heavy chain II variable region consists of the amino acid sequence set forth in SEQ ID NO: 14. In some embodiments, the heavy chain II comprises or consists of an amino acid sequence set forth in SEQ ID NO: 15 or SEQ ID NO: 17.

In some embodiments, the heavy chains comprise a heavy chain I targeting domain II of the HER2 protein and a heavy chain II targeting domain IV of the HER2 protein, wherein the heavy chain I and the heavy chain II are as described above.

In some embodiments, the anti-HER2 antibody is KN026 from Jiangsu Alphamab Biopharmaceuticals Co., Ltd. (hereinafter referred to as KN026).

In some embodiments, the cancer includes, but is not limited to, a hematological tumor or a solid tumor.

In some embodiments, the cancer is a hematological tumor. In some embodiments, the cancer is lymphoma or leukemia. In some embodiments, the lymphoma or leukemia includes, but is not limited to, myeloma, B-cell lymphoma, mantle cell lymphoma, non-Hodgkin B-cell lymphoma, non-Hodgkin T-cell lymphoma, cutaneous lymphoma, anaplastic large cell lymphoma, multiple myeloma, indolent non-Hodgkin lymphoma, plasmacytoma, chronic lymphocytic leukemia, small lymphocytic lymphoma, and follicular lymphoma. In some embodiments, the above cancers are recurrent or refractory.

In some embodiments, the cancer is a solid tumor. In some embodiments, the solid tumor includes, but is not limited to, bladder cancer, brain cancer, breast cancer, cervical cancer, chest tumor, endometrial cancer, esophageal squamous cell cancer, gastric cancer, head tumor, pancreatic cancer, bile duct cancer, colorectal cancer, eye cancer, head and neck squamous cell carcinoma, urothelial cancer, kidney cancer, liver cancer, lymph node cancer, lung cancer, oral cancer, neck tumor, ovarian cancer, prostate cancer, testicular cancer, laryngeal cancer and uterine cancer, melanoma, skin cancer, salivary gland cancer, soft tissue sarcoma, and osteosarcoma. In some embodiments, the above cancers are recurrent or refractory.

In some embodiments, the cancer is selected from breast cancer.

In some embodiments, the breast cancer is HER2-positive breast cancer, preferably IHC3+ HER2-positive breast cancer or IHC2+ and ISH-positive HER2-positive breast cancer. In some embodiments, the breast cancer is selected from hormone receptor-positive or -negative HER2-positive breast cancer, preferably HER2-positive breast cancer negative for both estrogen receptor and progestogen receptor, and HER2-positive breast cancer positive for estrogen receptor and/or progestogen receptor.

In some embodiments, the breast cancer includes: ductal carcinoma, lobular carcinoma, medullary carcinoma, colloid carcinoma, tubular carcinoma, inflammatory breast cancer, and triple-negative breast cancer (TNBC).

In some embodiments, the breast cancer is recurrent or metastatic breast cancer. In some embodiments, the breast cancer is selected from breast cancer with visceral metastasis and breast cancer without visceral metastasis.

In some embodiments, the breast cancer is recurrent and metastatic breast cancer.

In some embodiments, the breast cancer is recurrent or metastatic breast cancer that has not previously undergone systemic chemotherapy and/or HER2-targeted therapy for recurrent or metastatic breast cancer, or breast cancer that recurs 12 months or more after completion of neoadjuvant therapy or adjuvant therapy or HER2-targeted therapy. The systemic chemotherapy is selected from taxane chemotherapeutic medicaments such as docetaxel, albumin-bound paclitaxel, and capecitabine, vinorelbine, etc.; the HER2-targeted therapy is selected from trastuzumab, pertuzumab, pyrotinib, and ado-trastuzumab emtansine (tdm-1); the neoadjuvant treatment regimen is selected from: trastuzumab, taxanes, and pertuzumab combined with or not combined with carboplatin; and anthracyclines combined with cyclophosphamide and taxanes, trastuzumab, and pertuzumab; the adjuvant treatment regimen is selected from: anthracyclines combined with cyclophosphamide followed by taxanes combined with trastuzumab and pertuzumab; docetaxel combined with carboplatin combined with trastuzumab and pertuzumab; trastuzumab/pertuzumab followed by or not followed by neratinib; and tdm-1.

In some embodiments, the anti-HER2 antibody and the second therapeutic agent are the only therapeutically active ingredients.

In some embodiments, the use, method, or pharmaceutical composition or kit or kit of parts described herein further comprises a third therapeutic agent, wherein the third therapeutic agent is a chemotherapeutic agent. The chemotherapeutic agent is, for example, bendamustine, chlorambucil, cyclophosphamide, doxorubicin, vincristine, fludarabine, carboplatin, cisplatin, or any combination thereof.

In some embodiments, the use, method, or pharmaceutical composition or kit or kit of parts described herein further comprises a medicament for supportive therapy, e.g., granulocyte colony-stimulating factor (G-CSF).

In some embodiments, the anti-HER2 antibody and the second therapeutic agent are contained in the same formulation unit.

In some embodiments, the anti-HER2 antibody and the second therapeutic agent are contained in independent formulation units, respectively.

In some embodiments, the medicament, pharmaceutical composition, kit, or kit of parts described in the present application is administered to a patient or subject in need thereof in a clinically acceptable manner, preferably by intravenous infusion. Correspondingly, the anti-HER2 antibody and the second therapeutic agent are clinically acceptable formulations, preferably injection dosage forms, including liquid injections, powders for injection, and tablets for injection.

In some embodiments, the anti-HER2 antibody is formulated into a formulation, and each formulation unit has a content of the anti-HER2 antibody or a pharmaceutically acceptable form thereof (based on an anhydrous form of the anti-HER2 antibody) that is within a range of about 0.01 mg to about 400 mg, preferably about 300-400 mg, and more preferably about 300 mg, about 325 mg, about 350 mg, about 375 mg, or about 400 mg.

In some embodiments, the anti-HER2 antibody is administered at a dose of about 0.01 mg/kg-100 mg/kg, preferably about 20-40 mg/kg, about 20 mg/kg-30 mg/kg, or about 25 mg/kg-35 mg/kg, and more preferably about 30 mg/kg.

In some embodiments, the anti-HER2 antibody is administered in one or more administration cycles (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more administration cycles), preferably in three or more administration cycles. In some embodiments of the present disclosure, the administration cycles of the anti-HER2 antibody are continued until clinical benefits are lost (for example, disease progression, resistance, death, or unacceptable toxicity is confirmed).

In some embodiments, the docetaxel albumin composition is formulated into a formulation, and each formulation unit comprises 50-100 mg, preferably 70-90 mg, and more preferably about 80 mg, of docetaxel (based on anhydrous docetaxel).

In some embodiments, preferably, the docetaxel albumin composition is a powder for injection, and each vial of the powder for injection comprises about 50-100 mg, preferably about 70-90 mg, and more preferably about 80 mg of an active ingredient, based on anhydrous docetaxel.

In some embodiments, the docetaxel albumin composition is administered at a dose of about 60-125 mg/m², preferably about 60-100 mg/m², and more preferably about 60 mg/m², about 75 mg/m², or about 100 mg/m².

In some embodiments, the docetaxel albumin composition is administered in one or more administration cycles (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more administration cycles), preferably in three or more administration cycles. In some embodiments of the present disclosure, the administration cycles of the docetaxel albumin composition are continued until clinical benefits are lost (for example, disease progression, resistance, death, or unacceptable toxicity is confirmed).

In some embodiments of the present disclosure, each of the one or more administration cycles is 14 days in length. In some embodiments of the present disclosure, each of the one or more administration cycles is 21 days in length. In some embodiments of the present disclosure, each of the one or more administration cycles is 28 days in length. Preferably, each of the one or more administration cycles is 21 days in length.

In some embodiments of the present disclosure, the anti-HER2 antibody and/or the docetaxel albumin combination are/is administered QW (once weekly), Q2W (once every two weeks), Q3W (once every three weeks), or Q4W (once every four weeks).

In some embodiments of the present disclosure, the anti-HER2 antibody and/or the docetaxel albumin composition are/is administered once every 3 weeks (Q3W), with 21 days as one treatment cycle.

In some embodiments of the present disclosure, the anti-HER2 antibody and/or the docetaxel albumin composition are/is administered once every 4 weeks (Q4W), with 28 days as one treatment cycle.

In some embodiments of the present disclosure, the anti-HER2 antibody and/or the docetaxel albumin composition are/is administered once every 2 weeks (Q2W), with 14 days or 28 days as one treatment cycle.

In some embodiments of the present disclosure, the anti-HER2 antibody and/or the docetaxel albumin composition are/is administered once weekly (QW) for 3 consecutive weeks, followed by a one-week break, with 28 days as one treatment cycle.

In some embodiments of the present disclosure, the anti-HER2 antibody and/or the docetaxel albumin composition are/is administered once weekly (QW) for 2 consecutive weeks, followed by a one-week break, with 21 days as one treatment cycle.

In some embodiments of the present disclosure, the anti-HER2 antibody and/or the docetaxel albumin composition are/is administered on about day 1 (e.g., day 1, 2, 3, or 4) of each administration cycle.

In some embodiments of the present disclosure, the anti-HER2 antibody is administered before or after the docetaxel albumin composition is administered.

The medicament, pharmaceutical composition, kit, or kit of parts described in the present application is administered in a therapeutically effective amount to a patient or subject in need thereof to treat breast cancer. The breast cancer is as described above.

In some embodiments, the medicament, pharmaceutical composition, kit, or kit of parts of the present application comprises an anti-HER2 antibody and a docetaxel albumin composition and is administered in a therapeutically effective amount to a patient or subject in need thereof to treat breast cancer, wherein the breast cancer is as described above.

In some embodiments, the anti-HER2 antibody is administered once every 2-4 weeks, preferably once every 3 weeks, on day 1 of each cycle at a dose of about 20-40 mg/kg, preferably about 25-35 mg/kg, and more preferably about 30 mg/kg; the first intravenous drip infusion is administered over 90 min ± 15 min, and intravenous drip infusions in subsequent cycles are administered over 60 min ± 15 min. In some embodiments, the docetaxel albumin composition is administered once every 2-4 weeks, preferably once every 3 weeks, on day 1 of each cycle at a dose of about 60-125 mg/m², preferably about 60-100 mg/m², e.g., about 60 mg/m², about 75 mg/m², or about 100 mg/m², by intravenous drip infusion over 60 min ± 10 min.

In some embodiments, the anti-HER2 antibody is administered once every 3 weeks on day 1 of each cycle at a dose of about 30 mg/kg; the first intravenous drip infusion is administered over 90 min ± 15 min, and intravenous drip infusions in subsequent cycles are administered over 60 min ± 15 min. In some embodiments, the docetaxel albumin composition is administered once every 3 weeks on day 1 of each cycle at a dose of about 60 mg/m², about 75 mg/m², or about 100 mg/m² by intravenous drip infusion over 60 min ± 10 min.

In some embodiments, the docetaxel albumin composition described in the present application is prepared with reference to a method disclosed in WO2022184164A1. Studies have shown that both the suspension before lyophilization and the reconstituted suspension after lyophilization can remain stable for at least 24 hours at room temperature and for at least 10 days when refrigerated, without showing cloudiness or nanoparticle sedimentation. Compared to reconstituted suspensions of commercially available albumin-bound medicaments, which remain stable for only 8 h, this product significantly reduces the limitations on clinical use.

The docetaxel albumin composition described in the present application is a docetaxel albumin nanoparticle composition comprising docetaxel and an acid-denatured albumin and optionally comprising an osmotic pressure regulator and/or a pH regulator. The acid-denatured albumin is obtained by denaturing human serum albumin by adding an acid to achieve an appropriate pH value. Preferably:
(1) the acid is selected from acidic amino acids or acidic polypeptides, organic acids, and inorganic acids, wherein: the acidic amino acids or acidic polypeptides include, but are not limited to, cysteine hydrochloride, glutathione, etc.; the organic acids include, but are not limited to, citric acid, tartaric acid, etc.; the inorganic acids include, but are not limited to, hydrochloric acid, sulfuric acid, etc.; the acid is preferably cysteine hydrochloride, glutathione, or hydrochloric acid, and more preferably cysteine hydrochloride; and/or
(2) the appropriate pH value is preferably about 3.5-5.5, preferably about 3.5-5.0, more preferably about 3.8-4.7, and more preferably about 4.0-4.5; and/or
(3) based on anhydrous docetaxel, a mass ratio of docetaxel to human serum albumin is 1:(2.0-10.0), preferably 1:(3.0-7.0), more preferably 1:(4.0-6.0), and most preferably about 1:4.5; and/or
(4) the human serum albumin has a content of sodium octanoate of no greater than 0.08 mmol/g protein, preferably 0.03-0.08 mmol/g protein, further preferably 0.04-0.08 mmol/g protein, and more preferably 0.04-0.07 mmol/g protein.

In some embodiments, the docetaxel albumin nanoparticles have a particle size of about 60-200 nm, preferably about 90-150 nm, and more preferably about 90-135 nm. The type of the osmotic pressure regulator is not particularly limited. For example, the osmotic pressure regulator may be selected from sodium chloride, glucose, phosphate, citrate, etc., and is preferably sodium chloride. In some embodiments, the osmotic pressure regulator is sodium chloride, and a weight ratio of sodium chloride to docetaxel is (0.75-9):1, preferably (1-7):1, preferably (1.5-4.5):1, and most preferably about 2.25:1.

The type of the pH regulator is not particularly limited. The pH regulator is used for adjusting the pH value within an appropriate range. Preferably, the pH value range is 3.4-5.8, preferably 3.6-5.6 or 3.8-5.0, more preferably 3.9-4.8, and most preferably about 4.1.

There is no special requirement on whether the docetaxel in the docetaxel albumin composition described in the present application is in a hydrate form. The docetaxel may be selected from any one of anhydrous docetaxel, docetaxel hemihydrate, or docetaxel trihydrate; preferably, the docetaxel is selected from anhydrous docetaxel.

The content of the docetaxel described in the present application is based on anhydrous docetaxel.

The medicament described in the present application comprises the docetaxel albumin composition. The medicament is in a clinically acceptable dosage form, preferably an injection, and further preferably a liquid injection or a lyophilized powder for injection. For example, the docetaxel (albumin-bound) for injection used in specific embodiments is also referred to as DTX-HSA.

In some embodiments, the medicament is a liquid injection having a pH value of 3.4-5.8, preferably 3.6-5.6, or 3.8-5.0, and more preferably 3.9-4.8. The docetaxel albumin nanoparticles in the liquid injection have a particle size of about 60-200 nm, preferably about 90-150 nm, and more preferably about 90-135 nm; the liquid injection comprises 0-1.8% (w/v), preferably 0.45%-1.8% (w/v), more preferably 0.9%-1.8% (w/v), and more preferably 0.9% (w/v), sodium chloride. In some embodiments, the liquid injection comprises 2-10 mg/mL, preferably 2-8 mg/mL, docetaxel.

In some embodiments, the medicament is a lyophilized powder for injection comprising sodium chloride, and a weight ratio of sodium chloride to docetaxel is (0.75-9):1, preferably (1-7):1, preferably (1.5-4.5):1, and most preferably about 2.25:1. The docetaxel albumin nanoparticles have a particle size of about 60-200 nm, preferably about 90-150 nm, and more preferably about 90-135 nm. A suspension for preparing the lyophilized powder for injection has a pH of 3.4-5.8, preferably 3.6-5.6 or 3.8-5.0, more preferably 3.9-4.8, and most preferably about 4.1, before lyophilization.

The lyophilized powder for injection is reconstituted with a reconstitution medium to form a suspension before use. The reconstitution medium is selected from water for injection, a sodium chloride solution, and a glucose solution, preferably water for injection. The resulting reconstituted suspension has a pH value of 3.4-5.8, preferably 3.6-5.6 or 3.8-5.0, and more preferably 3.9-4.8. The docetaxel albumin nanoparticles in the reconstituted suspension have a particle size of about 60-200 nm, preferably about 90-150 nm, and more preferably about 90-135 nm. The reconstituted suspension comprises 0-1.8% (w/v), preferably 0.45%-1.8% (w/v), more preferably 0.9%-1.8% (w/v), and more preferably 0.9%, sodium chloride. In some embodiments, the reconstituted suspension comprises 2-10 mg/mL, preferably 2-8 mg/mL, docetaxel.

A preparation method for the docetaxel albumin composition described in the present application comprises the following steps:
(1) dissolving docetaxel in an organic solvent to obtain an organic-phase solution;
(2) taking a human serum albumin solution and adding an acid to adjust the pH value to obtain an acid-denatured albumin aqueous-phase solution; and taking another salt solution;
(3) mixing the organic-phase solution, the aqueous-phase solution, and the salt solution to load the drug to obtain a drug-loaded solution; and
(4) dialyzing to obtain a dialyzed suspension.

In the method, step (2) optionally comprises, before adding the acid to adjust the pH value, a step of diluting the human serum albumin solution with water for injection to obtain an albumin dilution.

In the method, step (2) optionally comprises, after adding the acid to adjust the pH value, an incubation step.

In the method, step (3) optionally comprises, after mixing to load the drug, a cooling step.

In the method, step (4) optionally comprises, before dialysis, concentrating the drug-loaded solution obtained in step (3) to obtain a concentrated solution.

In the method, step (4) optionally comprises, after dialysis, a concentration or dilution step to adjust the concentration of docetaxel in the post-dialysis suspension.

The method optionally comprises, after step (4), a step (5) of filtration for sterilization.

The method optionally comprises, after step (5), a step (6) of lyophilization.

In the method, the docetaxel described in step (1) is in any form, and is preferably anhydrous docetaxel, docetaxel hemihydrate, or docetaxel trihydrate. Based on anhydrous docetaxel, a mass ratio of docetaxel to human serum albumin is 1:(2.0-10.0), preferably 1:(3.0-7.0), more preferably 1:(4.0-6.0), and most preferably about 1:4.5).

In the method, the organic solvent in step (1) is selected from water-miscible solvents, such as ethanol, methanol, acetone, DMSO, etc., and is preferably ethanol. Based on anhydrous docetaxel, the organic-phase solution comprises 45-90 mg/mL, preferably 45-70 mg/mL, docetaxel.

In the method, the human serum albumin solution in step (2) has a content of sodium octanoate of no greater than 0.12 mmol/g protein, preferably no greater than 0.08 mmol/g protein, and preferably 0.045-0.08 mmol/g protein.

In the method, in step (2), the albumin dilution comprises 6-25 mg/mL, preferably 10-20 mg/mL, more preferably 12-18 mg/mL, and more preferably about 15 mg/mL, albumin.

In the method, in step (2), the acid is selected from acidic amino acids or acidic polypeptides, organic acids, and inorganic acids. The acidic amino acids or acidic polypeptides include, but are not limited to, cysteine hydrochloride, glutathione, etc.; the organic acids include, but are not limited to, citric acid, tartaric acid, etc.; the inorganic acids include, but are not limited to, hydrochloric acid, sulfuric acid, etc. The acid is preferably cysteine hydrochloride, glutathione, or hydrochloric acid, and more preferably cysteine hydrochloride. The pH value is preferably 3.5-5.5, preferably 3.5-5.0, more preferably 3.8-4.7, more preferably 4.0-4.5, and most preferably about 4.1.

In the method, in step (2), the incubation refers to heating to 35 °C-42 °C, preferably 38 °C-42 °C, after adding the acid to adjust the pH value; and incubating for 30 min or more, preferably 30 min-60 min.

In the method, the salt solution in step (2) is selected from aqueous solutions of sodium chloride, potassium chloride, sodium sulfate, and magnesium sulfate, and is preferably an aqueous solution of sodium chloride; the salt solution has a concentration of no less than 2%, preferably 2%-35%, and more preferably 10%-20%. In the method, in step (3), the drug loading conditions are heating the organic-phase solution and the aqueous-phase solution to 35 °C-42 °C, preferably 38 °C-42 °C, and mixing the three to load the drug.

In the method, the cooling in step (3) refers to cooling to below room temperature, preferably 0-20 °C, and more preferably 7-15 °C.

In the method, the concentrated solution in step (4) comprises about 4-10 mg/mL, preferably 6-10 mg/mL, more preferably 7-9 mg/mL, and more preferably 8 mg/mL, docetaxel.

In the method, the dialysis in step (4) is to remove excess small-molecule compounds; the type and amount of the dialysate and the molecular weight cutoff of the dialysis membrane are not particularly limited, and those skilled in the art can make selections based on general technical knowledge or experience. To facilitate further formulation and clinical use, the dialysate is preferably an aqueous solution of a clinically acceptable osmotic pressure regulator, such as an aqueous solution of sodium chloride, glucose, phosphate, or citrate. In some embodiments, in step (4), a sodium chloride solution is used as the dialysate for dialysis. The dialysis membrane has a molecular weight cutoff of 10-50 KDa, preferably 10-30 KDa, and more preferably 10 KDa or 30 KDa. The dialysate has a volume that is not less than 3 times, preferably 3-10 times, and more preferably 3-6 times, that of the drug-loaded solution or the concentrated solution. The sodium chloride solution has a concentration of no greater than 1.8% (w/v), preferably 0.45%-1.8% (w/v), and more preferably 0.9%-1.8% (w/v).

In some embodiments, the content of sodium octanoate in the human serum albumin solution used in step (2) needs to be adjusted in advance. Those skilled in the art can select a suitable method based on general technical knowledge or experience to adjust the content of sodium octanoate in the human serum albumin solution, including but not limited to dialysis.

In some embodiments, a method for adjusting the content of sodium octanoate in the human serum albumin solution is: taking a commercially available human serum albumin solution, diluting the solution with water for injection or normal saline to obtain a diluted albumin solution, and dialyzing the diluted albumin solution with water for injection or normal saline as the dialysate to partially remove sodium octanoate to obtain a human serum albumin solution with a low content of sodium octanoate.

In some embodiments, the human serum albumin solution is diluted by a factor of no less than 4, preferably 4-7. In the dialysis, the dialysis membrane has a molecular weight cutoff of 10-50 KDa, preferably 10-30 KDa, and more preferably 10 KDa or 30 KDa. The volume of the dialysate can be determined by those skilled in the art through routine tests based on the required content of sodium octanoate. Preferably, the dialysate has a volume that is about 3 times or more, preferably 3-10 times, and more preferably 3-6 times, that of the diluted albumin solution.

The human serum albumin solution with a low content of sodium octanoate has a content of sodium octanoate of less than 0.16 mmol/g protein, preferably less than 0.12 mmol/g protein, preferably less than 0.10 mmol/g protein, and preferably less than 0.08 mmol/g protein. The human serum albumin solution with a low content of sodium octanoate can be directly used for preparing the docetaxel albumin nanoparticle composition described in the present application, or can be mixed with a human serum albumin solution with another content of sodium octanoate in a ratio to obtain a desired content and then used for preparing the docetaxel albumin nanoparticle composition.

In the preparation method, excess small-molecule compounds will be removed from the drug-loaded solution or the concentrated solution through the dialysis step. For example, in some embodiments, in step (2), an acidic amino acid or acidic polypeptide is used to adjust the pH to prepare the acid-denatured albumin; the excess acidic amino acid or acidic polypeptide is substantially removed through the dialysis step; the composition is almost free of free acidic amino acid or acidic polypeptide molecules. The phrase "is almost free of free acidic amino acid or acidic polypeptide molecules" means that the content of free acidic amino acid or acidic polypeptide molecules in the composition is less than 0.25% (w/w) of docetaxel. For example, in some embodiments, cysteine hydrochloride or glutathione is used to adjust the pH to prepare the acid-denatured albumin, wherein the content of free cysteine or glutathione in the composition is less than 0.25% (w/w) of docetaxel.

The related sequences of the present disclosure are as follows:
CDR-L1 (Kabat) RASQDVNTAVA (SEQ ID NO: 1)
CDR-L2 (Kabat) SASFLYS (SEQ ID NO: 2)
CDR-L3 (Kabat) QQHYTTPPT (SEQ ID NO: 3)
VL 


(SEQ ID NO: 4)
LC 
DIQMTQSPSSLSASVGDRVTITCRASQDVNTAVAWYQQKPGKAPKLLIYSASFL
YSGVPSRFSGSRSGTDFTLTISSLQPEDFATYYCQQHYTTPPTFGQGTKVEIKRT
VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
(SEQ ID NO: 5)
CDR-H1-1 (Kabat) DYTMD (SEQ ID NO: 6)
CDR-H1-2 (Kabat) DVNPNSGGSIYNQRFKG (SEQ ID NO: 7)
CDR-H1-3 (Kabat) NLGPSFYFDY (SEQ ID NO: 8)
VH1 


 (SEQ ID NO: 9)
HC1 
EVQLVESGGGLVQPGGSLRLSCAASGFTFTDYTMDWVRQAPGKGLEWVADV
NPNSGGSIYNQRFKGRFTLSVDRSKNTLYLQMNSLRAEDTAVYYCARNLGPSF
YFDYWGQGTLVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDW
LNGKEYKCKVSNKALPAPIEKTISKAKGQPREPQVYTLPPSRDELTKNQVSLW
CLVKGFYPSDIAVEWESNGQPENNYKTTPPVLDSDGSFFLYSALTVDKSRWQQ
GNVFSCSVMHEALHNHYTQKSLSLSPGK (SEQ ID NO: 10)
CDR-H2-1 (Kabat) DTYIH (SEQ ID NO: 11)
CDR-H2-2 (Kabat) RIYPTNGYTRYADSVKG (SEQ ID NO: 12)
CDR-H2-3 (Kabat) WGGDGFYAMDY (SEQ ID NO: 13)
VH2 


 (SEQ ID NO: 14)
HC2 
EVQLVESGGGLVQPGGSLRLSCAASGFNIKDTYIHWVRQAPGKGLEWVARIY
PTNGYTRYADSVKGRFTISADTSKNTAYLQMNSLRAEDTAVYYCSRWGGDGF
YAMDYWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSN
TKVDKKVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSL
SCAVKGFYPSDIAVEWESNGQPENNYKTTPPVLDSDGSFKLVSKLTVDKSRWQ
QGNVFSCSVMHEALHNHYTQKSLSLSPGK (SEQ ID NO: 15)
HC1' 
EVQLVESGGGLVQPGGSLRLSCAASGFTFTDYTMDWVRQAPGKGLEWVADV
NPNSGGSIYNQRFKGRFTLSVDRSKNTLYLQMNSLRAEDTAVYYCARNLGPSF
YFDYWGQGTLVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDW
LNGKEYKCKVSNKALPAPIEKTISKAKGQPREPQVCTLPPSRDELTKNQVSLSC
 
AVKGFYPSDIAVEWESNGQPENNYKTTPPVLDSDGSFFLVSKLTVDKSRWQQG
NVFSCSVMHEALHNHYTQKSLSLSPGK (SEQ ID NO: 16)
HC2'

EVQLVESGGGLVQPGGSLRLSCAASGFNIKDTYIHWVRQAPGKGLEWVARIY
PTNGYTRYADSVKGRFTISADTSKNTAYLQMNSLRAEDTAVYYCSRWGGDGF
YAMDYWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSN
TKVDKKVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQD
WLNGKEYKCKVSNKALPAPIEKTISKAKGQPREPQVYTLPPCRDELTKNQVSL
WCLVKGFYPSDIAVEWESNGQPENNYKTTPPVLDSDGSFFLYSKLTVDKSRW
QQGNVFSCSVMHEALHNHYTQKSLSLSPGK (SEQ ID NO: 17)

It will be appreciated that within the scope of the present application, the above technical features of the present application and the technical features specifically described below (e.g., in the examples) may be combined to form new or preferred technical solutions. Due to space constraints, they are not described herein.

### BRIEF DESCRIPTION OF THE DRAWING

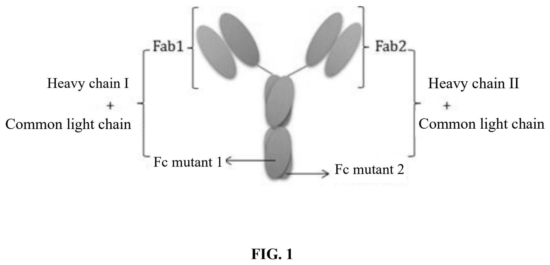

FIG. 1: a schematic diagram of the structure of KN026.

### DETAILED DESCRIPTION

The following examples are provided to better illustrate the present disclosure and should not be construed as limiting the present disclosure. Non-essential improvements and adjustments made by those skilled in the art to the embodiments based on the above description of the invention shall still fall within the scope of protection of the present disclosure.

As used herein, the singular forms "a", "an", and "the" also include plural forms, unless otherwise specified. For example, the term "a cell" includes a plurality of cells and mixtures thereof.

As used herein, the term "about" or "approximately" refers to the usual margin of error for the respective value readily known to those skilled in the art. Herein, the use of a value or parameter related to "about" or "approximately" includes (and describes) embodiments (or examples) involving the value or parameter itself.

As used herein, the term "contain", "comprise", or "include" is intended to be inclusive of the stated elements, integers, or steps, but not to exclude any other elements, integers, or steps. In the present disclosure, when the term "contain", "comprise", or "include" is used, unless otherwise indicated, it also encompasses the situation of consisting of the stated elements, integers, or steps. For example, when referring to an antibody variable region "comprising" a specific sequence, it is also intended to encompass an antibody variable region consisting of the specific sequence.

As used herein, the term "cancer" refers to a proliferative disorder disease caused by or characterized by cell proliferation, wherein the cells have lost susceptibility to normal growth control. The term "cancer" includes tumors and any other proliferative disorders. Cancers of the same tissue type originate in the same tissue and can be classified into different subtypes based on their biological characteristics.

As used herein, the term "treatment" refers to a clinical intervention intended to alter the natural course of disease of the individual or cell being treated during the course of clinical pathology. Desirable effects of treatment include delaying or decreasing the rate of disease progression, ameliorating or palliating the disease state, and remission or improved prognosis. For example, a patient is successfully "treated" if one or more symptoms associated with cancer are alleviated or eliminated, including but not limited to reducing the proliferation of cancerous cells (or destroying cancerous cells), decreasing symptoms resulting from the disease, improving the quality of life of the patient suffering from the disease, decreasing the dose of other medicaments required to treat the disease, delaying the progression of the disease, and/or prolonging the survival of the patient.

As used herein, the term "antibody" includes monoclonal antibodies (including full-length antibodies having an immunoglobulin Fc region), antibodies with multi-epitope specificity, multispecific antibodies (e.g., bispecific antibodies), dimers and single-chain antibodies, and antibody fragments (including antigen-binding fragments, such as Fab, F(ab')₂, and Fv).

As used herein, the term "CDR (complementarity determining region)" refers to amino acid sequences that together define the binding affinity and specificity of the Fv region of an antibody. The reason is that the specificity of an antibody lies in the structural complementarity between the antibody binding site and the antigenic determinant. The antibody binding site is composed of residues primarily from the so-called hypervariable regions or complementarity determining regions (CDRs). The light chain (L) and heavy chain (H) of an antibody each have three CDRs. The antigen-binding site of a conventional antibody includes six CDRs comprising CDR sets from each of the heavy chain variable region and the light chain variable region.

As used herein, the term "heavy chain variable region (VH)" refers to the amino-terminal variable region domain of an immunoglobulin heavy chain.

As used herein, the term "light chain variable region (VL)" refers to the amino-terminal variable region domain of an immunoglobulin light chain.

As used herein, the term "formulation unit" refers to the smallest subpackage of each pharmaceutical formulation comprising an appropriate amount of an active compound. For example, a unit formulation in capsules refers to one capsule, a unit formulation in tablets refers to one tablet, and a unit formulation in injections refers to one injection; this applies to other types of formulations.

As used herein, the term "objective response rate (ORR)" is defined as the percentage of participants who achieve complete response (CR) or partial response (PR) during the study, as per the RECIST 1.1 criteria.

As used herein, the term "partial response" or "PR" means that there is at least a decrease of ≥30% in the sum of the diameters (SOD) of the target lesions compared to the baseline, non-target lesions show no progression, and no new lesions are formed, as per the RECIST 1.1 criteria.

As used herein, the term "complete response" or "CR" means that all non-lymph-node target lesions and non-target lesions disappear, and no new lesions are formed, as per the RECIST 1.1 criteria.

As used herein, the term "therapeutically effective amount" refers to an amount that produces a therapeutic effect on a subject. For example, in a subject to which the amount is administered, the symptoms or states of a disease are mitigated, palliated, or eliminated, or the progression of the symptoms or states of a disease is delayed or inhibited, compared to a subject to which the amount is not administered.

As used herein, the term "intolerant" means that a subject has previously received anticancer therapy, but experienced adverse event symptoms (e.g., peripheral neuropathy, skin toxicity, hyperglycemia, and gastrointestinal symptoms) in the first two cycles; despite adherence to dose adjustment guidelines, the symptoms showed no improvement, leading to treatment discontinuation, and there was no evidence for disease progression.

As used herein, the term "CnDm" refers to day m of the n^{th} administration cycle; for example, C1D1 refers to the time when the first dose is administered, and C4D1 refers to day one of the fourth administration cycle.

As used herein, the term "recurrent" means the occurrence of a new lesion after cancer (e.g., breast cancer) has previously undergone radical therapy, which indicates the recurrence of cancer (e.g., breast cancer).

As used herein, the term "metastatic" refers to metastasis in which the site affected by a tumor lesion is beyond sites such as ipsilateral supraclavicular lymph nodes of the primary site, ipsilateral internal mammary lymph nodes of the primary site, ipsilateral axillary lymph nodes of the primary site, the ipsilateral chest wall of the primary site, and the skin of the primary site, which is distant metastasis.

In some embodiments, the anti-HER2 antibody consists of two different heavy chains (H) and two common light chains (L) with identical sequences. Each of the common light chains has three CDRs, designated CDR-L1, CDR-L2, and CDR-L3. In some embodiments, the common light chains comprise a CDR-L1 set forth in SEQ ID NO: 1, a CDR-L2 set forth in SEQ ID NO: 2, and a CDR-L3 set forth in SEQ ID NO: 3. The heavy chains comprise a heavy chain I targeting domain II of the HER2 protein and a heavy chain II targeting domain IV of the HER2 protein. The heavy chain I has three CDRs, designated CDR-H1-1, CDR-H1-2, and CDR-H1-3. In some embodiments, the heavy chain I comprises a CDR-H1-1 set forth in SEQ ID NO: 6, a CDR-H1-2 set forth in SEQ ID NO: 7, and a CDR-H1-3 set forth in SEQ ID NO: 8. The heavy chain II has three CDRs, designated CDR-H2-1, CDR-H2-2, and CDR-H2-3. In some embodiments, the heavy chain II comprises a CDR-H2-1 set forth in SEQ ID NO: 11, a CDR-H2-2 set forth in SEQ ID NO: 12, and a CDR-H2-3 set forth in SEQ ID NO: 13. The heavy chain I and the heavy chain II in the present application are only used to distinguish two heavy chains targeting different epitopes, and do not have sequential or positional meanings.

The docetaxel albumin composition described in the present application is also referred to as docetaxel (albumin-bound). In some embodiments, the docetaxel albumin composition is a docetaxel albumin nanoparticle composition prepared using a conventional method for preparing an albumin nanoparticle medicament in the art. As human serum albumin is an endogenous substance in the human body, it has good biocompatibility. It can be used as a natural carrier of hydrophobic drugs, increase the solubility of poorly soluble drugs, and utilize the distinctive "gp60-caveolin-SPARC" channel of albumins to enrich tumor regions with drugs, thereby improving efficacy.

In some embodiments, the docetaxel albumin composition described in the present application is prepared with reference to a method disclosed in WO2022184164A1, the disclosure of which is incorporated herein by reference in its entirety.

In some embodiments, the anti-Her2 bispecific antibody disclosed in the present application is KN026. The sequences of the two common light chains of KN026 are both the amino acid sequence set forth in SEQ ID NO: 5, the sequence of the heavy chain I is the amino acid sequence set forth in SEQ ID NO: 10, and the sequence of the heavy chain II is the amino acid sequence set forth in SEQ ID NO: 15. The structure of KN026 is shown in FIG. 1. The antibody KN026 can be prepared using conventional technical means in the art.

The Fab1 (heavy chain I) of the antibody protein KN026 can specifically recognize domain II of human HER2 protein and block signaling resulting from heterodimer formation between HER2 and other ERBB family members, and its Fab2 (heavy chain II) targets domain IV of human HER2 protein to inhibit HER2-related signaling pathways. The two inhibitory means act synergistically, so that the bispecific antibody KN026 can more effectively inhibit the proliferation of HER2-positive tumor cells. In addition, the CH3 domains of the two heavy chains of KN026 comprise a range of asymmetric amino acid mutations for increasing the yield of heterodimeric antibody proteins while still retaining full Fc functionality. KN026 has significant antibody-dependent cell-mediated cytotoxicity. The results of *in vitro* anti-tumor cell proliferation experiments show that the above antibody inhibits tumor cell proliferation in a dose-dependent manner. In some embodiments, the KN026 and related formulations thereof disclosed in the present application can be prepared using conventional methods in the art. For example, the formulations may be liquid formulations. For example, the formulations may be prepared in aqueous carriers. For example, a stabilizer may be added in an amount that is not greater than the amount that may result in an undesirable viscosity or a viscosity unsuitable for intravenous administration. For example, the liquid formulations may further comprise one or more of a buffer, a surfactant, and a preservative.

### Preparation Example

The docetaxel (albumin-bound) for injection used in the example was produced and provided by CSPC Group, and was prepared with reference to the method for formula 1-1 of Example 1 of Patent No. WO2022184164A1. Specifically:
(1) 8 g of anhydrous docetaxel was weighed out and dissolved in 120 mL of ethanol to obtain an organic-phase solution.
(2) A human serum albumin solution containing 36 g of the albumin (with a content of sodium octanoate of 0.08 mmol/g protein) was diluted with water for injection to obtain a solution containing 15 mg/mL albumin. Then, an appropriate amount of cysteine hydrochloride was added to adjust the pH to 4.1, and the solution was incubated at 42 °C for 30 min to obtain an acid-denatured albumin aqueous-phase solution. 18 g of sodium chloride was dissolved in water for injection to prepare a 20% salt solution.
(3) The organic-phase solution and the aqueous-phase solution were heated to 42 °C. The organic-phase solution, the aqueous-phase solution, and the salt solution were mixed to load the drug, and the resulting material was cooled in an ice-water bath to 15 °C to obtain a drug-loaded solution.
(4) The drug-loaded solution was concentrated until the concentration of docetaxel was about 8 mg/mL, to obtain a concentrated solution. The concentrated solution was 5-fold dialyzed with an isotonic sodium chloride solution (0.9%, w/v) as the dialysate and a dialysis membrane molecular weight cutoff of 30 KDa to obtain a post-dialysis suspension. Then, an appropriate amount of the dialysate was added to adjust the concentration of docetaxel in the suspension to 4 mg/mL.
(5) The suspension was sterilized by filtration using 0.45 µm + 0.2 µm membranes to obtain a suspension before lyophilization.
(6) The suspension before lyophilization obtained in step (5) was lyophilized to obtain a lyophilized powder.

KN026 comprises a heavy chain I, a heavy chain II, and common light chains. The amino acid sequence of the heavy chain I is set forth in SEQ ID NO: 10, the amino acid sequence of the heavy chain II is set forth in SEQ ID NO: 15, and the amino acid sequences of the common light chains are set forth in SEQ ID NO: 5.

Cells were transfected with a vector containing nucleic acid sequences encoding the heavy chain I, the heavy chain II, and the common light chains described above. After expression and purification, KN026 was obtained. For the specific preparation process, refer to Patent Application No. WO2016110267.

### Example 1: Clinical Study

This is a randomized, controlled, open-label, multicenter, superiority phase III clinical study intended to compare the effectiveness and safety of KN026 + docetaxel (albumin-bound) for injection versus the trastuzumab + pertuzumab + docetaxel first-line therapy in the treatment of subjects with HER2-positive recurrent or metastatic breast cancer.

### 1. Study method

### 1.1. Investigational medicaments and administration regimens:

### (1) Investigational medicaments

| **Name/code number of medicament** | **Active ingredient** | **Strength** | **Storage conditions** |
|---|---|---|---|
| **Experimental group** | | | |
| KN026 | Recombinant humanized anti-HER2 bispecific antibody | 325 mg, 13 mL/vial | Stored at 2-8 °C in a dark place |
| Docetaxel for injection (albumin-bound) | Docetaxel albumin composition | 80 mg/vial | Stored below 25 °C |

| **Control group** | | | |
|---|---|---|---|
| Pertuzumab injection | Pertuzumab | 420 mg/vial | Stored at 2-8 °C in a dark place |
| Trastuzumab for injection | Trastuzumab | 150 mg/vial or 60 mg/vial | Stored at 2-8 °C in a dark place |
| Docetaxel injection | Docetaxel | 0.5 mL: 20 mg/injection | Stored at 2-25 °C in a dark place |

The investigational medicaments were all provided by CSPC Group (Shanghai JMT-BIO Technology Co.,Ltd ). (2) Administration regimen for experimental group 1) KN026

Administered once every 3 weeks on day 1 of each cycle at a dose of 30 mg/kg. The first intravenous drip infusion is administered over 90 min (± 15 min). If no infusion reactions occur, the infusion time for subsequent cycles may be adjusted to 60 min (± 15 min). Each vial contains 325 mg (13 mL) of the antibody KN026. The required medicament volume is calculated based on body weight. The corresponding volume of the medicament is withdrawn and diluted into 250 mL of normal saline to obtain a dilution for intravenous drip infusion.

### 2) Docetaxel (albumin-bound) for injection

Administered once every 3 weeks on day 1 of each cycle at a dose of 100 mg/m² by intravenous drip infusion over 60 min (± 10 min).

Each vial contains 80 mg of docetaxel (albumin-bound) powder for injection. The powder is dispersed and dissolved in 20 mL of water for injection to form a 4 mg/mL suspension. The corresponding administration volume is calculated based on the subject's body surface area. This volume of the suspension is injected into an infusion bag, and normal saline (its volume is the same as the volume described above) is then added for dilution, resulting in a 2 mg/mL solution for intravenous drip infusion.

For the experimental group, the medicaments are administered in the following order: KN026 and docetaxel (albumin-bound) for injection. An observation period of at least 30 min is recommended between administrations of the two medicaments.

To prevent severe hematological toxicity, prophylactic use of granulocyte colony-stimulating factor (G-CSF) is permitted. G-CSF should be used 24-72 h after completing the infusion of docetaxel (albumin-bound) for injection.

### (3) Administration regimen for control group:

### 1) Pertuzumab injection

Administered once every 3 weeks on day 1 of each cycle. The starting dose is 840 mg and is administered by intravenous infusion over 60 min (± 10 min). In each subsequent cycle, the dose is 420 mg, and the infusion time is 30-60 min (± 10 min). Note: If a delayed dose results in an interval of ≥6 weeks between two consecutive infusions, a loading dose of 840 mg of pertuzumab should be re-administered, and subsequently, pertuzumab is administered at a dose of 420 mg once every 3 weeks.

Each vial contains 14 mL of a concentrated solution with a concentration of 30 mg/mL. The corresponding administration volume is calculated, and this volume of the concentrated solution is added to 250 mL of normal saline to form a dilution for intravenous drip infusion. Note: Do not dilute pertuzumab with 5% glucose solution, as it is chemically and physically unstable in 5% glucose solution.

### 2) Trastuzumab for injection

Administered once every 3 weeks on day 1 of each cycle. The starting dose is 8 mg/kg and is administered by intravenous infusion over 90 min (± 10 min). In each subsequent cycle, the dose is 6 mg/kg and is administered by intravenous infusion over 30-90 min (± 10 min). If a delayed dose results in an interval of ≥6 weeks between two consecutive infusions, a loading dose of 8 mg/kg trastuzumab should be re-administered, and subsequently, trastuzumab is administered at a dose of 6 mg/kg once every 3 weeks.

The corresponding administration volume is calculated based on the subject's body weight, and this volume of the medicament is added to 250 mL of normal saline to form a dilution for intravenous drip infusion.

### 3) Docetaxel injection

Administered once every 3 weeks on day 1 of each cycle at a dose of 75 mg/m², with each infusion administered over a period of no less than 60 min.

Prior to the administration of docetaxel injection, pretreatment is required, as per the package insert [one day before docetaxel injection, oral administration of a glucocorticoid such as dexamethasone at 16 mg per day (e.g., twice daily, 8 mg each time) for 3 days] or clinical routine practice.

For the control group, the medicaments are administered in the following order: pertuzumab, trastuzumab, and docetaxel. An observation period of at least 30 min is recommended between administrations of every two medicaments.

### (4) Dose adjustment

A dose reduction is not permitted for KN026, trastuzumab, or pertuzumab in this study. When a treatment-related adverse event occurs with docetaxel (albumin-bound) for injection or docetaxel injection, a dose reduction is permitted. For docetaxel (albumin-bound) for injection, a maximum of two dose reductions are permitted. If a third reduction is required, treatment should be discontinued permanently. For docetaxel injection, only one dose reduction is permitted. If a second reduction is required, treatment should be discontinued permanently.

| | **Docetaxel (albumin-bound) for injection** | **Docetaxel injection** |
|---|---|---|
| Initial dose | 100 mg/m² | 75 mg/m² |
| First reduction | 75 mg/m² | 60 mg/m² |
| Second reduction | 60 mg/m² | Not applicable |

### 1.2. Inclusion and exclusion criteria

### Inclusion criteria

Subjects must meet all of the following inclusion criteria to be eligible for enrollment in this study:
1) Voluntarily participate in this study and sign the informed consent form (ICF).
2) Age ≥ 18 years.
3) Histologically and/or cytologically confirmed recurrent or metastatic breast cancer.
4) A test on the most recent tumor tissue sample proved HER2-positive (IHC3+, or IHC2+ and ISH-positive), as confirmed by the central laboratory.
5) No prior systemic chemotherapy and/or HER2-targeted therapy for recurrent or metastatic breast cancer (subjects who have received one endocrine treatment regimen are allowed to be enrolled). Subjects who experienced recurrence 12 months or more after completing (neo)adjuvant chemotherapy or HER2-targeted therapy may be considered for enrollment.
6) Eastern Cooperative Oncology Group (ECOG) performance status score of 0-1.
7) Presence of at least one measurable or evaluable lesion (RECIST 1.1).
8) Adequate organ and bone marrow function (without blood transfusion or use of hematopoietic stimulating factor medicaments for correction within 14 days prior to testing):
   a) Neutrophil count ≥ 1.5 × 10⁹/L;
   b) Platelet count ≥ 100 × 10⁹/L;
   c) Hemoglobin ≥ 90 g/L;
   d) Liver function: total bilirubin (TBIL) ≤ 1.0 × upper limit of normal (ULN); alanine aminotransferase (ALT) and aspartate aminotransferase (AST) ≤ 2.5 × ULN (≤ 5 × ULN for subjects with liver metastases);
   e) Kidney function: creatinine clearance ≥ 30 mL/min (calculated using the Cockcroft-Gault formula);
   f) Blood coagulation: international normalized ratio (INR) ≤ 1.5 and activated partial thromboplastin time (APTT) ≤ 1.5 × ULN (for subjects who are receiving anticoagulant therapy, the investigator must judge that INR and APTT are both within a safe and effective therapeutic range);
   g) Left ventricular ejection fraction (LVEF) > 50%.
9) Expected survival time ≥ 3 months.
10) Female subjects of childbearing potential must have a negative blood pregnancy test result within 7 days prior to randomization and agree to use reliable and effective contraception methods during the study treatment period and within 3 months (docetaxel injection and docetaxel (albumin-bound) for injection) or 7 months (trastuzumab, pertuzumab, and KN026) after the last dose of study treatment, whichever is the longest; male subjects with female partners of childbearing potential must agree to use reliable and effective contraception methods during the study treatment period and within 3 months (docetaxel injection and docetaxel (albumin-bound) for injection) or 7 months (trastuzumab, pertuzumab, and KN026) after the last dose of study treatment, whichever is the longest.

### Exclusion criteria

Subjects who meet any one of the following criteria will be excluded from this study:
1) Those who have contraindications to trastuzumab, pertuzumab, and docetaxel or are deemed by the investigator to be unsuitable for receiving KN026 or docetaxel (albumin-bound) for injection.
2) ALT and/or AST > 1.5 × ULN concurrent with alkaline phosphatase (ALP) > 2.5 × ULN.
3) Subjects with grade ≥3 peripheral neuropathy at the time of randomization.
4) Toxicities from prior antitumor therapy have not recovered to Common Terminology Criteria for Adverse Events (CTCAE 5.0) grade ≤1 (except for toxicities determined by the investigator to pose no safety risk, such as alopecia, peripheral neuropathy, and isolated laboratory tests, which must be alleviated to grade ≤2).
5) Those who experienced LVEF dropping to <50%, or symptomatic congestive heart failure (CHF), or related toxicities that led to permanent discontinuation of treatment, during prior anti-HER2 medicament therapy.
6) Those who have known allergies and/or contraindications (including but not limited to active digestive tract ulcer, severe hypertension, severe hypokalemia, glaucoma, etc.) to glucocorticoids.
7) Those who have previously received anthracycline medicament therapy reaching the following cumulative doses: doxorubicin or liposomal doxorubicin > 360 mg/m²; epirubicin > 720 mg/m²; mitoxantrone > 120 mg/m²; other anthracycline medicaments > an amount equivalent to 360 mg/m² doxorubicin. If more than one anthracycline medicament was used, the cumulative dose must not exceed an amount equivalent to 360 mg/m² doxorubicin.
8) Use of strong inhibitors of CYP3A4 within 14 days prior to randomization.
9) Contraindication or history of hypersensitivity to any study medicament component or any known excipient.
10) Untreated or unstable brain parenchymal metastases, spinal cord metastases or compression, or carcinomatous meningitis. Subjects who have received local brain therapy, have stable symptoms, have stable imaging test results for at least 4 weeks prior to randomization, have no evidence of cerebral edema, and do not require glucocorticoid treatment may be considered for enrollment.
11) Active malignancies within 3 years prior to randomization, except for the breast cancer studied in this trial and any locally curable tumors that have undergone radical therapy (e.g., resected basal cell or squamous cell skin cancer, superficial bladder cancer, cervical or breast cancer *in situ,* and early thyroid cancer).
12) Uncontrolled or significant cardiovascular or cerebrovascular diseases, including but not limited to:
   a) New York Heart Association (NYHA) class II or higher congestive heart failure, unstable angina, myocardial infarction, or arrhythmia causing hemodynamic instability, within 6 months prior to randomization;
   b) Primary cardiomyopathy (e.g., dilated cardiomyopathy, hypertrophic cardiomyopathy, arrhythmogenic right ventricular cardiomyopathy, restrictive cardiomyopathy, or unclassified cardiomyopathy);
   c) History of clinically significant QT interval prolongation, or QTcF (calculated using the Fridericia formula) > 450 ms during screening;
   d) Arterial/venous thrombotic events within 6 months prior to randomization, such as cerebrovascular accidents (including transient ischemic attack, cerebral hemorrhage, and cerebral infarction), deep vein thrombosis, and pulmonary embolism; and
   e) Uncontrolled hypertension (systolic blood pressure > 160 mmHg and/or diastolic blood pressure > 100 mmHg).
13) Severe chronic or active infections (including tubercle bacillus infection, etc.) requiring systemic antibacterial, antifungal, or antiviral therapy within 14 days prior to randomization.
14) Active hepatitis B (hepatitis B surface antigen positive and HBV-DNA ≥ 2 × 10³ IU/mL) or hepatitis C (hepatitis C antibody positive and the quantitative HCV-RNA test result > the lower limit of detection). Note: For hepatitis B virus carriers with the lower limit of detection < HBV DNA < 2 × 10³ IU/mL, subjects who are willing to receive entecavir or other antiviral therapy based on clinical judgments during the study may be considered for enrollment.
15) Those who have a known history of human immunodeficiency virus (HIV) infection.
16) Those who are currently participating in another clinical study (except for non-interventional clinical studies or follow-up phases of interventional studies), or the interval between randomization and the end (last dose) of the previous clinical study is less than 4 weeks.
17) Antitumor therapy within 28 days prior to randomization, including radiotherapy, targeted therapy, immunotherapy, other clinical study medicaments, etc., or use of traditional Chinese medicines with antitumor indications within 14 days prior to randomization.
18) Uncontrolled serous cavity effusion (e.g., pleural effusion, abdominal effusion, or pericardial effusion) requiring frequent drainage or medical intervention within 7 days prior to randomization, or need for additional intervention (excluding exfoliative cytology of effusion) within 2 weeks after intervention.
19) Major organ surgery (excluding needle biopsy) within 28 days prior to randomization.
20) Pregnant or breastfeeding women.
21) Other situations that may interfere with the subject's procedure for participating in the study, or are not in line with the maximum benefit of the subject's participation in the study, or affect study results: a history of a disease such as psychosis, drug abuse, substance abuse, or the like, any other clinically significant diseases or conditions, etc.

### 1.3. Evaluation indices

### 1.3.1. Efficacy evaluation

The imaging efficacy evaluation used RECIST 1.1. The efficacy evaluation indices included:
ORR: The proportion of subjects achieving CR and subjects achieving PR among all subjects (evaluated by BIRC and the investigator).
(PR: There is a decrease of ≥30% in the sum of the diameters of the target lesions compared to the baseline sum of the diameters of the target lesions, non-target lesions show no progression, and no new lesions are formed.
CR: All non-lymph-node target lesions and non-target lesions disappear, and no new lesions are formed.)

Periodic safety evaluations were performed throughout the study, including physical examinations, vital signs, body weight, 12-lead electrocardiogram, echocardiogram, laboratory tests, etc. The severity of adverse events was recorded using the National Cancer Institute's Common Terminology Criteria for Adverse Events, Version 5.0 (NCI-CTCAE 5.0).

### 2. Study results

(1) The efficacy evaluation results are shown below:
   As of February 2024, a total of 267 subjects had been enrolled. Among them, 170 had undergone post-baseline efficacy evaluations, with 85 in the experimental group and 85 in the control group (trastuzumab + pertuzumab + docetaxel). Among the 85 subjects in the experimental group, 67 achieved PR, resulting in an ORR of 78.8%. Among the 85 subjects in the control group, 56 achieved PR, resulting in an ORR of 65.9%. Both the PR and ORR values of the experimental group were significantly greater than those of the control group, indicating that at this stage, the ORR data of the experimental group of the present application achieved a more favorable antitumor efficacy trend compared to the control group.
(2) The safety evaluation results are shown below:
   As of now, no new adverse reaction signals have been found in the experimental group of the present application.

The combination of the anti-HER2 antibody (KN026) and the docetaxel albumin composition has superior antitumor efficacy against HER2-positive recurrent or metastatic breast cancer compared to existing standard treatment regimens, has good overall safety, and is more convenient to administer. The combination can provide such patients with a first-line treatment option that has superior efficacy and good safety and is more convenient to administer.

## Claims

1. Use of an anti-HER2 antibody and a second therapeutic agent in the preparation of a medicament for treating cancer, wherein the second therapeutic agent is a docetaxel albumin composition.

2. Use of an anti-HER2 antibody in the preparation of a medicament for enhancing efficacy of a second therapeutic agent against cancer, wherein the second therapeutic agent is a docetaxel albumin composition.

3. A method for treating cancer with an anti-HER2 antibody and a second therapeutic agent, wherein the second therapeutic agent is a docetaxel albumin composition.

4. A pharmaceutical composition or kit or kit of parts for treating cancer, comprising an anti-HER2 antibody and a second therapeutic agent, wherein the second therapeutic agent is a docetaxel albumin composition.

5. The use, method, or pharmaceutical composition or kit or kit of parts according to any one of claims 1-4, wherein the anti-HER2 antibody is an anti-HER2 bispecific antibody; preferably, the anti-HER2 antibody is a bispecific antibody targeting different epitopes of human HER2 protein; more preferably, the different epitopes of the HER2 protein are domain II and domain IV thereof.

6. The use, method, or pharmaceutical composition or kit or kit of parts according to any one of claims 1-5, wherein the anti-HER2 antibody comprises two common light chains with identical sequences; preferably, the common light chains comprise a CDR-L1 set forth in SEQ ID NO: 1, a CDR-L2 set forth in SEQ ID NO: 2, and a CDR-L3 set forth in SEQ ID NO: 3; more preferably, the common light chains comprise a light chain variable region set forth in SEQ ID NO: 4; further preferably, the common light chains comprise an amino acid sequence set forth in SEQ ID NO: 5.

7. The use, method, or pharmaceutical composition or kit or kit of parts according to any one of claims 1-6, wherein the anti-HER2 antibody comprises two heavy chains with different sequences; preferably, the heavy chains comprise a heavy chain I targeting domain II of the HER2 protein and/or a heavy chain II targeting domain IV of the HER2 protein.

8. The use, method, or pharmaceutical composition or kit or kit of parts according to claim 7, wherein the heavy chain I comprises a CDR-H1-1 set forth in SEQ ID NO: 6, a CDR-H1-2 set forth in SEQ ID NO: 7, and a CDR-H1-3 set forth in SEQ ID NO: 8; preferably, the heavy chain I comprises a heavy chain I variable region set forth in SEQ ID NO: 9; more preferably, the heavy chain I comprises an amino acid sequence set forth in SEQ ID NO: 10 or SEQ ID NO: 16; and/or
the heavy chain II comprises a CDR-H2-1 set forth in SEQ ID NO: 11, a CDR-H2-2 set forth in SEQ ID NO: 12, and a CDR-H2-3 set forth in SEQ ID NO: 13; preferably, the heavy chain II comprises a heavy chain II variable region set forth in SEQ ID NO: 14; more preferably, the heavy chain II comprises an amino acid sequence set forth in SEQ ID NO: 15 or SEQ ID NO: 17.

9. The use, method, or pharmaceutical composition or kit or kit of parts according to any one of claims 1-8, wherein the docetaxel albumin composition comprises docetaxel and an acid-denatured albumin; preferably, the docetaxel is selected from anhydrous docetaxel; more preferably, a weight ratio of the docetaxel to the albumin is 1:(4.0-6.0).

10. The use, method, or pharmaceutical composition or kit or kit of parts according to claim 9, wherein the acid-denatured albumin is obtained by denaturing human serum albumin by adding an acid to achieve an appropriate pH value; preferably, the acid is cysteine hydrochloride; preferably, the pH value is about 4.1.

11. The use, method, or pharmaceutical composition or kit or kit of parts according to any one of claims 1-10, wherein the docetaxel albumin composition further comprises an osmotic pressure regulator and sodium octanoate; preferably, the osmotic pressure regulator is sodium chloride; more preferably, a weight ratio of sodium chloride to the docetaxel is about 2.25:1; preferably, a content of sodium octanoate is less than 0.16 mmol/g protein; more preferably, the content of sodium octanoate is less than 0.08 mmol/g protein.

12. The use, method, or pharmaceutical composition or kit or kit of parts according to any one of claims 1-11, wherein the cancer includes, but is not limited to, a hematological tumor or a solid tumor.

13. The use, method, or pharmaceutical composition or kit or kit of parts according to claim 12, wherein the hematological tumor is lymphoma or leukemia, including but not limited to myeloma, B-cell lymphoma, mantle cell lymphoma, non-Hodgkin B-cell lymphoma, non-Hodgkin T-cell lymphoma, cutaneous lymphoma, anaplastic large cell lymphoma, multiple myeloma, indolent non-Hodgkin lymphoma, plasmacytoma, chronic lymphocytic leukemia, small lymphocytic lymphoma, and follicular lymphoma; the solid tumor includes, but is not limited to, bladder cancer, brain cancer, breast cancer, cervical cancer, chest tumor, endometrial cancer, esophageal squamous cell cancer, gastric cancer, head tumor, pancreatic cancer, bile duct cancer, colorectal cancer, eye cancer, head and neck squamous cell carcinoma, urothelial cancer, kidney cancer, liver cancer, lymph node cancer, lung cancer, oral cancer, neck tumor, ovarian cancer, prostate cancer, testicular cancer, laryngeal cancer and uterine cancer, melanoma, skin cancer, salivary gland cancer, soft tissue sarcoma, and osteosarcoma.

14. The use, method, or pharmaceutical composition or kit or kit of parts according to claim 12, wherein the solid tumor is breast cancer; preferably, the breast cancer is HER2-positive breast cancer; more preferably, the breast cancer is HER2-positive recurrent and/or metastatic breast cancer.

15. The use, method, or pharmaceutical composition or kit or kit of parts according to any one of claims 1-14, wherein the anti-HER2 antibody and the second therapeutic agent are contained in the same formulation unit, or in independent formulation units, respectively.

16. The use, method, or pharmaceutical composition or kit or kit of parts according to any one of claims 1-15, wherein the docetaxel albumin composition is formulated into a formulation, and each formulation unit comprises 50-100 mg, preferably 70-90 mg, and more preferably about 80 mg, of docetaxel (based on anhydrous docetaxel); and/or the anti-HER2 antibody is formulated into a formulation, and each formulation unit has a content of the anti-HER2 antibody or a pharmaceutically acceptable form thereof (based on an anhydrous form of the anti-HER2 antibody) that is within a range of about 0.01 mg to about 400 mg, preferably 300-400 mg, and more preferably about 300 mg, about 325 mg, about 350 mg, about 375 mg, or about 400 mg.

17. The use, method, or pharmaceutical composition or kit or kit of parts according to any one of claims 1-16, wherein the docetaxel albumin composition is administered at a dose of 60-125 mg/m², preferably 60-100 mg/m², and more preferably about 60 mg/m², about 75 mg/m², or about 100 mg/m²; and/or the anti-HER2 antibody is administered at a dose of 0.01 mg/kg-100 mg/kg, preferably 20-40 mg/kg, 20 mg/kg-30 mg/kg, or 25 mg/kg-35 mg/kg, and more preferably about 30 mg/kg.
